# EUROPEAN PATENT SPECIFICATION

(11) **EP 4 606 230 B1**
(45) Date of publication and mention of the grant of the patent: **04.11.2026**
(21) Application number: 24220341.2
(22) Date of filing: 16.12.2024
(51) Int. Cl.: A23L 33/115, A61K 9/107

(54) **FORMULATION COMPRISING MILK FAT GLOBULE-BASED NANOCARRIERS FOR THE DELIVERY OF ACTIVE AGENTS AND METHOD TO OBTAIN THE SAME**
FORMULIERUNG MIT NANOTRÄGERN AUF MILCHFETTKÜGELCHENBASIS ZUR FREISETZUNG VON WIRKSTOFFEN UND VERFAHREN ZUR HERSTELLUNG DAVON
FORMULATION COMPRENANT DES NANOVECTEURS À BASE DE GLOBULE GRAS DU LAIT POUR L'ADMINISTRATION D'AGENTS ACTIFS ET SON PROCÉDÉ D'OBTENTION

(30) Priority: 20.02.2024 PT 2024119268; 12.11.2024 PT 2024119828
(43) Date of publication of application: 27.08.2025
(73) Proprietor: Universidade Do Porto, 4099-002 Porto (PT); REQUIMTE - Rede de Química e Tecnologia, 4050-453 Porto (PT)
(72) Inventor: RODRIGUES, MARIA, 4050-313 PORTO (PT); COSTA, JOÃO, 4050-313 PORTO (PT); NUNES, CLÁUDIA, 4050-313 PORTO (PT); SOARES, FILIPA, 4050-313 PORTO (PT); GRANJA, ANDREIA, 4050 PORTO (PT); CABRITA, ANA, 4050-313 PORTO (PT); FONSECA, ANTÓNIO, 4050 PORTO (PT)
(74) Representative: Couto, Cláudia

(56) References cited:
- CN-A- 117 257 732
- US-A1- 2021 290 538
- LINHAN ZHANG: "Transparent Dispersions of Milk Fat-Based Solid Lipid Nanoparticles for Delivery of beta-Carotene", MASTER THESIS, 1 August 2013 (2013-08-01), Knoxville, U.S.A., pages 1 - 98, XP055478358, Retrieved from the Internet <URL:http://trace.tennessee.edu/cgi/viewcontent.cgi?article=2841&context=utk_gradthes> [retrieved on 20180525]

## Description

### Technical field

The present invention relates to a formulation comprising milk fat globule-based nanocarriers and a method to produce the same.

### Background art

Many active agents and food additives have some limitations such as short shelf-life, poor gastrointestinal stability or low bioavailability, which considerably reduces their applicability. Nanotechnology-based approaches have been considered to attempt to overcome this issue and have already shown great potential, primarily in medical applications [1-6] but also in the food industry for human [7-10] and both monogastric and ruminant animal [11, 12] nutrition. However, these nano-delivery systems also have their limitations, such as biocompatibility of the used components, complex production methods that compromise the scalability or even reduced efficacy [13-17].

Milk is one of the world's most consumed foods and is used in countless applications in food technology, with some of its constituents having even been considered for nanotechnology-based applications with promising results already reported. Milk components are widely regarded as safe and already used in countless applications in food technology, making them ideal candidates for nanodelivery system components due to their inherent biocompatibility [18-21]. In fact, some milk constituents such as caseins or naturally occurring exosomes have already been proposed for nanotechnology-based applications with promising results reported [10, 22-24]. Milk fat, on the other hand, also has tremendous potential for producing nanoparticles [19, 25] but has so far been less used. Milk fat is usually separated from raw milk to produce skimmed milk and either discarded or used to produce butter, an increasingly less common practice due to the rise of alternative products. The milk fat layer, or milk cream, obtained in this way is not however composed solely of lipids but also has around 5-10% of protein and some lactose. The existing milk fat-based nanoparticle studies focus only on the fat content [26, 27], primarily anhydrous milk fat, which not only requires additional processing to obtain but also removes the proteins associated with the milk fat globules that can play important roles in their absorption.

Formulations for the delivery of actives that comprise milk fat are also known from e.g., US 2021/290538 A1; and Linhan Zhang,"Transparent dispersions of milk-fat based solid lipid nanoparticles for delivery of beta-carotene", Master thesis, 1 August 2013, Knoxville, USA.

This creates an opportunity for the valorisation of a sometimes-wasted by-product through the development of a highly biocompatible nanodelivery system for oral applications [19, 28]. Additionally, milk fat's high lipid composition (around 85% lipids) enables nanocarriers to be produced using the same methods as lipid nanoparticles, which are among the most easily scalable [11, 15, 29].

### Summary

The present application relates to a formulation comprising milk fat globule-based nanocarriers for the delivery of active agents, comprising:
- between 60% to 66% of lipid content on a dry basis m/m;
- between 4% to 5% of protein content on a dry basis m/m;
- between 23% to 27% of a non-ionic surfactant on a dry basis m/m;
and further comprises at least one type of active agent encapsulated in the nanocarriers.

In one embodiment, the active ingredient, or mixtures thereof, is present between 5% to 20% on a dry basis m/m.

In one embodiment, it further comprises between 6% to 8% of lactose on a dry basis m/m.

In one embodiment, at least one active agent is selected from bioactive compounds, nutraceuticals, therapeutic agents, dietary supplements, or mixtures thereof.

In one embodiment, the lipid content comprises lipids selected from short-chain saturated fatty acids, short-chain unsaturated fatty acids, long-chain saturated fatty acids, long-chain unsaturated fatty acids, triacylglycerols, diacylglycerols, monoacylglycerols, phosphatidylcholine, phosphatidylethanolamine, phosphatidylinositol, phosphatidylserine, glycerophospholipids, phospholipids, sphingomyelin, sphingolipids, cholesterol, or mixtures thereof.

In one embodiment, the protein content comprises proteins selected from mucin, xanthine dehydrogenase/oxidase, butyrophilin, cluster of differentiation CD36, Periodic Acid Schiff 6/7, Periodic Acid Schiff III, adipophilin, fatty-acid binding proteins, immunoglobulins, proteins derived from the cytoplasm of secretory-epithelial cells, skim milk constituents, proteins from milk leucocytes, or mixtures thereof.

In one embodiment, the nanocarriers comprise 75% to 90% nanocarriers of an average particle size between 160 and 220 nm, and 10% to 25% of an average particle size between 500 and 600 nm.

In one embodiment, the nanocarriers have an average particle size between 80 and 140 nm.

In one embodiment, the nanocarriers have an average -25 mV to -35 mV Zeta potential.

In one embodiment, the milk fat is obtained from milk of a mammal selected from bovine, dairy cow, goat, sheep, donkey, camel, human, or mixtures thereof.

In one embodiment, the formulation is for oral, topical, transdermal or intravenous administration.

In one embodiment, the formulation is used in a food product, as nutritional supplement, in enteral or parenteral assisted feeding, in a cosmetic composition, or in a pharmaceutical composition.

The application also relates to a method to obtain the milk fat globule-based nanocarriers, comprising the following steps:
- Obtaining milk from a mammal source;
- Centrifuging the milk between 13000 and 15000 xg for a time between 27 and 33 min at a temperature between 4 and 20 ⁰C, to separate the milk cream from the remaining milk components;
- Freezing the milk cream overnight between -75 and -85 °C;
- Lyophilizing the milk cream for a time between 10 to 20 h at a temperature between -70 to -85 °C under a pressure between 500 to 20000 Pa, to obtain milk fat;
- Adding the milk fat and a non-ionic surfactant in a ratio between 2.5:1 and 3.5:1 of milk fat:surfactant, in a water bath;
- Adding an active agent, or mixtures thereof, in a proportion between 1:5 and 1:20 of active agent:milk fat+surfactant;
- Heating the mixture at a temperature between 45 and 55 °C for a time between 10 and 15 mins;
- Adding double deionised water heated at the same temperature in a proportion between 18:1 and 20:1 of water:dry components, to produce an emulsion; and:
   - Sonicating the emulsion for a time between 4 and 6 min at 75% and 85% amplitude, to form a nanoemulsion;
   - Cooling the nanoemulsion to a temperature between 20 and 25°C, to obtain an aqueous suspension comprising the nanocarriers;
      or
   - Homogenizing the emulsion for a time between 4 and 6 mins at between 11000 and 13000 rpm,
   - Keeping the emulsion in a water bath at a temperature between 45 and 55 °C;
   - Transferring the emulsion to a syringe and placing it in a high-pressure homogenization chamber and homogenizing at a pressure between 340 and 360 MPa and temperature between 45 and 55 °C;
- Collecting the resulting nanoemulsion and allowing to cool at a temperature between 20 and 25°C, to obtain an aqueous suspension comprising the nanocarriers.

In one embodiment, the non-ionic surfactant is selected from Tween60, Tween80, polyvinyl alcohol, Poloxamers, Fatty acid esters of glycerol or sorbitan, lecithin, saponins or Amino acid-based surfactants.

### General description

The present application relates to innovative milk fat-based nanocarriers obtained from dairy industry by-products as building blocks.

The formulation comprising milk fat globule-based nanocarriers herein described is made of biocompatible nanocarriers obtained from milk fat globules primarily separated from raw milk, for example bovine milk which is the most available and commonly consumed milk in Western society, and non-ionic surfactants already used in the pharmaceutical and food industries. Studies were also performed using goat/caprine milk instead of bovine milk, to assess the impacts of the different lipid profile.

Furthermore, the milk fat globule-based nanocarriers (MiGloN) are obtained with a simple and easily scalable method with equipment already widely used in industrial settings, greatly facilitating the large-scale production and enabling applications either in medicine or food technology.

Contrary to the approaches in the prior art until now, the use of the entire milk cream, or milk fat globules, requires less processing when compared to other approaches and also does not discard the proteins associated with the milk fat globules' membrane that further stabilize the nanocarrier structure but can also bring benefits regarding their uptake in the target subject.

The formulation comprising milk fat globule-based nanocarriers presents promising physical properties, high biocompatibility, inherent antioxidant activity and considerable bioactive encapsulation potential.

The formulation comprising milk fat globule-based nanocarriers remained stable over 10 months of storage at room temperature (20-25°C). Additionally, they were able to resist digestion in both gastric and intestinal mimicking conditions highlighting their potential for an oral supplementation approach. Furthermore, the novel nanocarriers formulation also didn't present any cytotoxicity up to 2.5 mg/mL and were found to be nonhemolytic, confirming their biosafety considering the existing legislation for food and biomedical devices.

Several active agents were successfully encapsulated within the nanocarriers: folic acid, vitamin D, vitamin E and DHA; and the nanocarriers themselves exhibited antioxidant effects.

Two organic-solvent-free production methods were tested: one based on sonication and another on high-pressure homogenization (HPH). The sonication-based method was the main method used for the nanocarrier characterization, whereas the high-pressure homogenization method was mainly applied to prove the suitability of large-scale production methods for the formulation comprising milk fat globule-based nanocarriers.

In the methods herein disclosed, the milk fat layer (or cream) is separated from the milk by centrifugation. This fat layer is then freeze-dried to remove all the water, leaving only the solid components of the milk cream. The freeze-dried fat layer is then mixed with a non-ionic surfactant and heated to melt the lipids present therein. Water (heated to the same temperature as the lipids) is added to the fat layer and surfactant mixture to produce an oilin-water emulsion, which is afterwards ultrasonicated or homogenized at high pressure to generate a nanoemulsion that, when cooled, will become an aqueous dispersion of nanocarriers.

Active agents can be added, at different moments of the production depending on their properties, to be encapsulated within the nanocarrier thus improving their stability, resistance, biocompatibility and/or bioavailability.

All results allow to state that the formulation comprising milk fat globule-based nanocarriers is an exceptional candidate for active agent delivery applications, with inherent antioxidant activity, in both medical and food sectors.

The nanocarriers can then be consumed to provide health and/or nutritional benefits to the consumer. They can be incorporated into existing food products, be used as nutritional supplements or even in enteric or parenteric assisted feedings. Additionally, these nanocarriers could also be applied in cosmetic or pharmaceutical products and be orally, transdermally, intravenously, or topically administered or even be delivered in a parenteral manner.

### Brief description of drawings

For easier understanding of this application, figures are attached that represent the preferred forms of implementation which nevertheless are not intended to limit the technique disclosed herein.

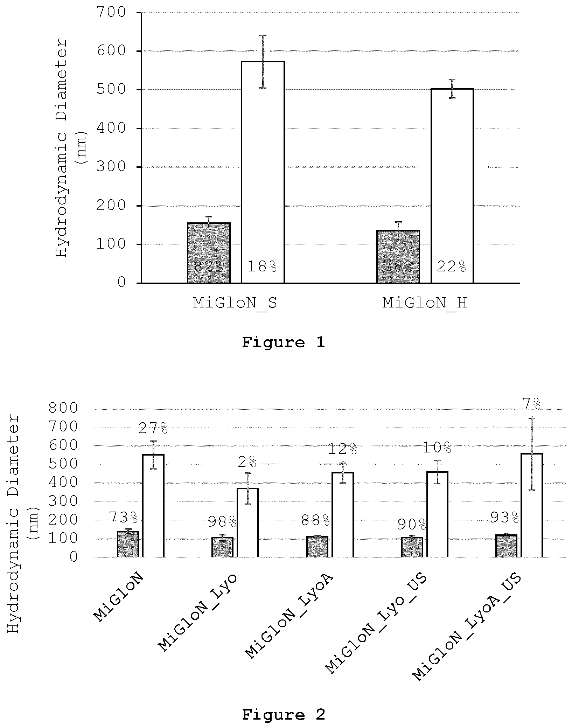

Figure 1 shows the dynamic light scattering results obtained for milk fat globule-based nanocarriers produced by sonication (MiGloN_S) and high-pressure homogenization (MiGloN_H) methods. Results regarding diameter, different shades indicate different populations and % values indicate the abundance of that population, shown as mean ± standard deviation, n = 3. No statistical differences were found between the two production methods (p < 0.05).
Figure 2 shows the dynamic light scattering results obtained for milk fat globule-based nanocarriers produced with bovine milk fat layer via the sonication-based method after: production (MiGloN), lyophilization (MiGloN_Lyo), lyophilization with 1% AEROSIL^{®} (MiGloN_LyoA), lyophilization and resuspended using ultrasounds (MiGloN_Lyo_US) and lyophilization with 1% aerosil and resuspended using ultrasounds (MiGloN_LyoA_US). Results regarding diameter, different shades indicate different populations and % values indicate the abundance of that population, shown as mean ± standard deviation, n = 3. No statistical differences were found between any of the lyophilization strategies and the original nanocarriers (p < 0.05).

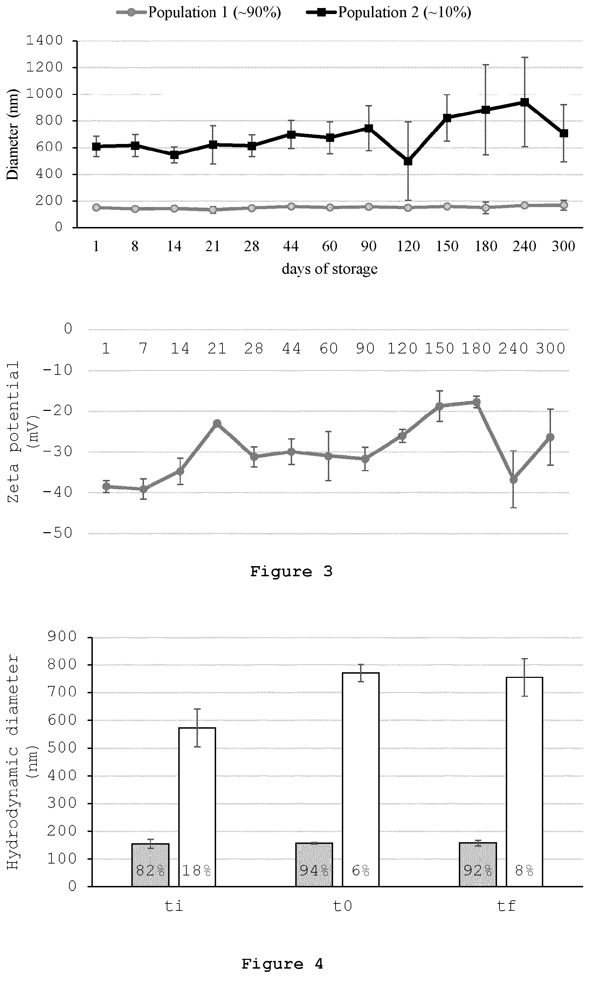

Figure 3 shows the dynamic light scattering and zeta potential results obtained for milk fat globule-based nanocarriers, produced with bovine milk fat layer via the sonication-based method, over a storage period of 300 days, at room temperature. Results regarding diameter, different shades indicate different populations and % values indicate the abundance of that population; polydispersity index (PdI) and zeta potential shown as mean ± standard deviation, n = 3. No statistical differences were found between any of the timepoints and the original nanocarriers (p < 0.05).
Figure 4 shows the dynamic light scattering results obtained for milk fat globule-based nanocarriers (MiGloN), produced with bovine milk fat layer via the sonication-based method, after: production (ti), contact with gastric medium (t0) and 2 h incubation in gastric medium (tf). Results regarding diameter, different shades indicate different populations and % values indicate the abundance of that population, shown as mean ± standard deviation, n = 3. No statistical differences were found between any of the timepoints or the initial nanocarriers (p < 0.05).

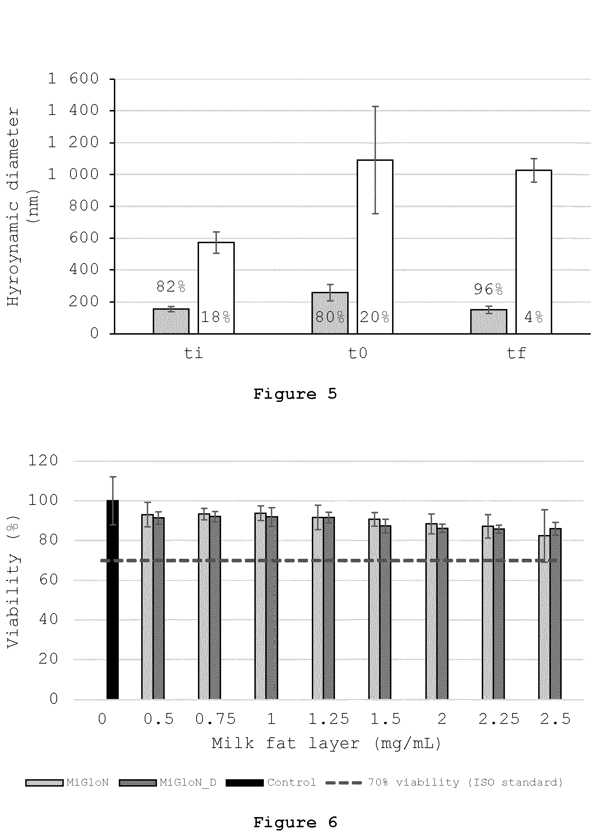

Figure 5 shows the dynamic light scattering results obtained for milk fat globule-based nanocarriers, produced with bovine milk fat layer via the sonication-based method, after: production (ti), contact with intestinal medium (t0) and 4 h incubation in intestinal medium (tf). Results regarding diameter, different shades indicate different populations and % values indicate the abundance of that population, shown as mean ± standard deviation, n = 3. * denotes statistical differences from the initial nanocarriers, no statistical differences were found between the two incubation time points (p < 0.05).
Figure 6 shows the cellular viability assay with L929 fibroblast cells exposed to milk fat globule-based nanocarriers, produced with bovine milk fat layer via the sonication-based method, with (MiGloN_D) and without (MiGloN) vitamin D and at increasing concentrations (0.5, 0.75, 1, 1.25, 1.5, 2, 2.25 and 2.5 mg of milk fat per mL of medium). The recommended threshold value of 70% is indicated in the graph as a dotted line. Values shown as mean ± standard deviation, n = 3. No statistical differences were found between the control and any of nanocarriers at any concentration (p < 0.05).

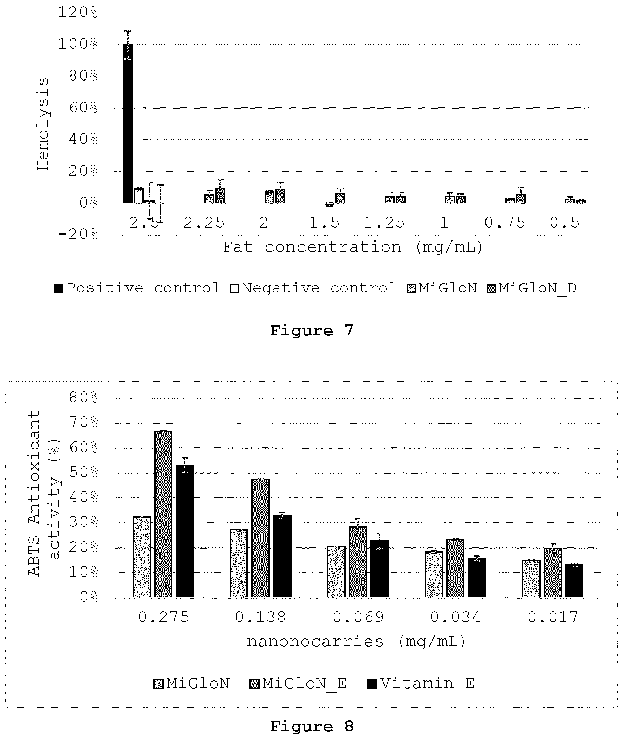

Figure 7 shows the hemolysis assay results performed using fresh human red blood cells exposed to milk fat globule-based nanocarriers, produced with bovine milk fat layer via the sonication-based method, with (MiGloN_D) and without (MiGloN) vitamin D and at increasing concentrations (0.5, 0.75, 1, 1.25, 1.5, 2, 2.25 and 2.5 mg of milk fat per mL of medium). Values shown as mean ± standard deviation, n = 3. No statistical differences were found between the negative control and any of nanocarriers at any concentration and the positive control was significantly different from any other condition (p < 0.05).
Figure 8 shows the ABTS radical scavenging assay results for milk fat globule-based nanocarriers, produced with bovine milk fat layer via the sonication-based method, with (MiGloN_E) and without (MiGloN) vitamin E and free vitamin E. Increasing concentrations (0.02, 0.03, 0.07, 0.14 and 0.28 mg of nanocarriers per mL of medium) were used and the free vitamin E was calculated to have the same amount of vitamin E as that encapsulated in the MiGloN. Values shown as mean ± standard deviation, n = 3.

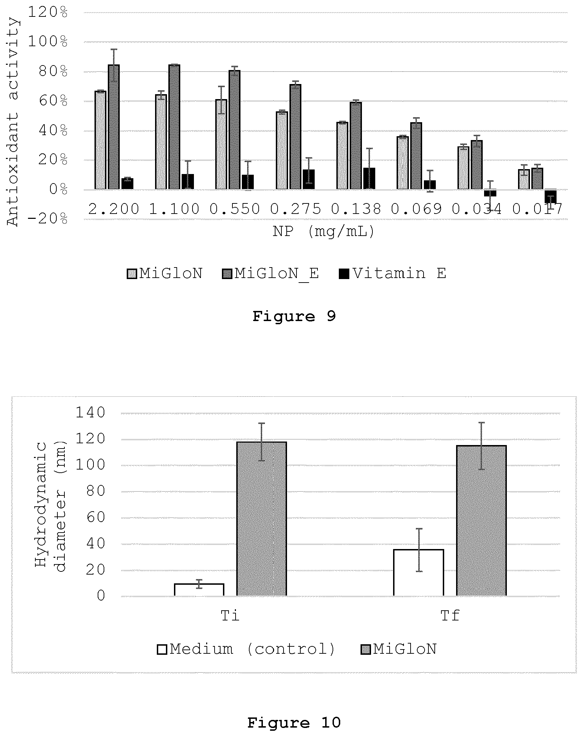

Figure 9 shows the Oxygen Radical Absorbance Capacity (ORAC) assay results for milk fat globule-based nanocarriers, produced with bovine milk fat layer via the sonication-based method, with (MiGloN_E) and without (MiGloN) vitamin E and free vitamin E. Increasing concentrations (0.017, 0.034, 0.069, 0.138, 0.275, 0.550, 1.100 and 2.200 mg of nanocarriers prr mL of medium) were used and the free vitamin E was calculated to have the same amount of vitamin E as that encapsulated in the MiGloN. Values shown as mean ± standard deviation, n = 3.
Figure 10 shows the dynamic light scattering results obtained for milk fat globule-based nanocarriers, produced with caprine milk fat layer via the sonication-based method, as well as controls of only ruminal medium after: contact with ruminal fluid (t0) and 24 h incubation in ruminal fluid medium (tf). Results regarding diameter, different shades indicate different populations, shown as mean ± standard deviation, n = 3. No statistical differences were found between the two incubation time points (p < 0.05).

### Detailed description of embodiments

Now, preferred embodiments of the present application will be described in detail with reference to the annexed drawings. However, they are not intended to limit the scope of this application.

The present application discloses a formulation comprising milk fat globule-based nanocarriers for the delivery of active agents.

In one embodiment, the formulation comprises at least one type of active agent encapsulated in the nanocarriers.

In the context of the present invention, active agents are understood as chemical or biological substances or molecules that have a chemical or biological effect on living organisms, tissues, or cells.

In one embodiment, an active agent can be selected from bioactive compounds, nutraceuticals, therapeutic agents, dietary supplements or mixtures thereof.

In one embodiment, such active agents can be, for example, therapeutic agents suitable to treat, prevent or ameliorate a medical condition in a subject or nutraceutical agents, such as, but not limited to, phytochemicals, vitamins, flavonoids, or natural antioxidants, which are suitable to develop functional foods that, when consumed, will not only improve nutrition but also provide additional health benefits.

In one embodiment, the active agents can be selected from, but not limited to, vitamins, flavonoids, polyphenols, sensitive nutrients or medical drugs with low bioavailability.

In one embodiment, the formulation comprising milk fat globule-based nanocarriers is obtained from milk of a mammal such as, but not limited to, bovine, dairy cow, goat, sheep, donkey, camel, human, or mixtures thereof. Furthermore, the production of milk fat globule-based nanocarriers was possible using milk collected from different mammals, of different species, at different production stages and fed different diets. It was found that the milk source did not substantially affect the final properties and characteristics of the nanocarriers. The nanocarriers formulation obtained from different milk sources had similar characteristics and properties.

In one embodiment, the milk can comprise lactose. In another embodiment, the milk can be lactose free.

The formulation comprising milk fat globule-based nanocarriers suitable for the delivery of active agents comprises:
- between 60% to 66% of lipid content on a dry basis m/m;
   and
- between 4% to 5% of protein content on a dry basis m/m;
- between 23% to 27% of a non-ionic surfactant on a dry basis m/m;
and further comprises at least one type of active agent encapsulated in the nanocarriers,

In one embodiment, the active ingredient, or mixtures thereof, are present in between 5% to 20% on a dry basis m/m.

The formulation is also 90% to 95% composed of water, making it an aqueous formulation.

In one embodiment, the formulation can further comprise between 6% to 8% of lactose on a dry basis m/m.

Both the lipid and protein composition can change depending on the mammal source and their diet, but it can contain any component of the milk's fat globules or their associated membrane. These include, but are not limited to, lipid content comprises lipids selected from short-chain saturated fatty acids, short-chain unsaturated fatty acids, long-chain saturated fatty acids, long-chain unsaturated fatty acids, triacylglycerols, diacylglycerols, monoacylglycerols, phosphatidylcholine, phosphatidylethanolamine, phosphatidylinositol, phosphatidylserine, glycerophospholipids, phospholipids, sphingomyelin, sphingolipids, cholesterol, or mixtures thereof.

In one embodiment, the protein content is selected from, but not limited to, mucin (MUC1), the redox enzyme xanthine dehydrogenase/oxidase (XDH/XO), butyrophilin (BTN), cluster of differentiation (CD36), Periodic Acid Schiff 6/7 (PAS 6/7), Periodic Acid Schiff III (PAS III), adipophilin (ADPH), fatty-acid binding proteins (FABP), immunoglobulins, proteins derived from the cytoplasm of secretory-epithelial cells, skim milk constituents, proteins from milk leucocytes, or mixtures thereof.

In one embodiment, the formulation comprises milk fat globule-based nanocarriers with 80% to 90% of nanocarriers with of an average particle size between 160 and 220 nm, and 10% to 20% of an average particle size between 500 and 600 nm. This data was obtained for the nanocarriers obtained with the sonication method and using bovine milk fat layer.

In one embodiment, the formulation comprises milk fat globule-based nanocarriers with 75% to 90% of nanocarriers with an average particle size between 160 and 220 nm, and 10% to 25% of an average particle size between 500 and 600 nm. This data was obtained for the nanocarriers obtained with the HPH method and using bovine milk fat layer.

In one embodiment, the formulation comprises milk fat globule-based nanocarriers with an average particle size between 80 and 140 nm. This data was obtained for the nanocarriers obtained with the sonication method and using caprine milk fat layer.

In one embodiment, the formulation comprises milk fat globule-based nanocarriers having an average -25 mV to -35 mV Zeta potential. This data was obtained for the nanocarriers obtained with either the sonication or the HPH method and using bovine or caprine milk fat layer.

In one embodiment, the formulation comprising milk fat globule-based nanocarriers has a shelf-life of at least 300 days. This data was obtained for the nanocarriers obtained with the sonication method and using bovine milk fat layer.

In one embodiment, the formulation comprising milk fat globule-based nanocarriers has a shelf-life of at least 210 days. This data was obtained for the nanocarriers obtained with the sonication method and using caprine milk fat layer.

In one embodiment, the formulation comprising milk fat globule-based nanocarriers is stable for at least 2 hours in gastric mimetic medium. This data was obtained for the nanocarriers obtained with the sonication method and using bovine milk fat layer.

In one embodiment, the formulation comprising milk fat globule-based nanocarriers is stable for at least 4 hours in intestinal mimetic medium. This data was obtained for the nanocarriers obtained with the sonication method and using bovine milk fat layer.

In one embodiment, the formulation comprising milk fat globule-based nanocarriers is stable for at least 24 hours in ruminal *ex vivo* medium. This data was obtained for the nanocarriers obtained with the sonication method and using caprine milk fat layer.

In one embodiment, the formulation comprising milk fat globule-based nanocarriers has up to 100% encapsulation potential, when considering vitamin D3, vitamin E or DHA. This data was obtained for the nanocarriers obtained with the sonication method and using bovine milk fat layer. When considering vitamin D, similar results were obtained for the nanocarriers obtained with the sonication method and using caprine milk fat layer.

The above-mentioned characteristics and properties (particle size, zeta potential, stability, encapsulation potential, etc.) of the formulation comprising milk fat globule-based nanocarriers can be consolidated as an average because they are consistent across the milk sources and method used to produce the nanocarriers.

In one embodiment, the formulation comprising milk fat globule-based nanocarriers can be administered orally, topically, transdermally or intravenously.

In one embodiment, the formulation comprising milk fat globule-based nanocarriers can be used in a food product, as nutritional supplement, in enteral or parenteral assisted feeding, in a cosmetic composition, or in a pharmaceutical composition.

In general, the formulation comprising milk fat globule-based nanocarriers has also shown to have no cytotoxicity, no hemolytic activity, but has antioxidant capacity.

In another aspect of the invention, a sonication method to obtain the milk fat globule-based nanocarriers comprises the following steps:
- Obtaining milk from a mammal source;
- Centrifuging the milk between 13000 and 15000 xg for a time between 27 and 33 min at a temperature between 4 and 20 ⁰C, to separate the milk cream from the remaining milk components;
- Freezing the milk cream overnight between -75 and -85 °C;
- Lyophilizing the milk cream for a time between 10 to 20 h at a temperature between -70 to -85 °C under a pressure between 500 to 20000 Pa, to obtain milk fat;
- Adding the milk fat and a non-ionic surfactant in a ratio between 2.5:1 and 3.5:1 of milk fat:surfactant, in a water bath;
- Adding an active agent, or mixtures thereof, in a proportion between 1:5 and 1:20 of active agent:milk fat+surfactant;
- Heating the mixture at a temperature between 45 and 55 °C for a time between 10 and 15 mins;
- Adding double deionised water heated at the same temperature in a proportion between 18:1 and 20:1 of water:dry components, to produce an emulsion;
- Sonicating the emulsion for a time between 4 and 6 min at 75% and 85% amplitude, to form a nanoemulsion;
- Cooling the nanoemulsion to a temperature between 20 and 25°C, to obtain an aqueous suspension comprising the nanocarriers.

In another aspect of the invention, a high-pressure homogenization method to obtain the milk fat globule-based nanocarriers comprises the following steps:
- Obtaining milk from a mammal source;
- Centrifuging the milk at 13000 and 15000 xg for a time between 27 and 33 min at a temperature 4 and 20 ⁰C, to separate the milk cream from the remaining milk components;
- Freezing the milk cream overnight at a temperature between -75 and -85 °C;
- Lyophilizing the milk cream for a time between 10 to 20 h at a temperature between -70 to -85 °C under a pressure between 500 to 20000 Pa, to obtain milk fat;
- Adding milk fat and a non-ionic surfactant in a ratio between 2.5:1 and 3.5:1 for milk fat:surfactant, in a water bath;
- Adding an active agent, or mixtures thereof, in a proportion between 1:5 and 1:20 of active agent:milk fat+surfactant;
- Heating the mixture at a temperature between 45 and 55 °C for a time between 10 and 15 mins;
- Adding double deionised water heated at the same temperature in a proportion between 18:1 and 20:1 for water:dry components, to produce an emulsion;
- Homogenizing the emulsion for a time between 4 and 6 mins at between 11000 and 13000 rpm,
- Keeping the emulsion in a water bath at a temperature between 45 and 55 °C;
- Transferring the emulsion to a syringe and placing it in an HPH chamber and homogenizing at a pressure between 340 and 360 MPa and temperature between 45 and 55 °C;
- Collecting the resulting nanoemulsion and allowing to cool at a temperature between 20 and 25°C, to obtain an aqueous suspension comprising the nanocarriers.

Both methods follow common steps and diverge in the step to obtain the nanoemulsion, optionally selecting a sonication step or a HPH step.

In one embodiment of the HPH method, between 8 and 9 mL of the emulsion are transferred to a syringe.

In one embodiment, the active agent, or mixtures thereof, is added in a proportion between 1:5 and 1:20 (active agent:milk fat+surfactant).

The point at which the active agent is added in the method must take into account its hydro-lipophilic characteristics and its stability under production conditions (e.g. exposure to oxygen-containing atmospheres), namely, for example, before the lipid and surfactant melting, after this melt or dissolved in the aqueous phase.

In one embodiment, the non-ionic surfactant is selected from, but not limited to, Tween60 (polysorbate monostearate), Tween80 (polysorbate monooleate), polyvinyl alcohol, Poloxamers, Fatty acid esters of glycerol or sorbitan, lecithin, saponins, or Amino acid-based surfactants.

In one embodiment, the active agent can be selected from, but not limited to, folic acid, vitamin D, vitamin E or DHA (a long chain polyunsaturated fatty acid - LC-PUFA), of mixtures thereon.

In the context of the present invention, room temperature (RT) is generally understood to be between 20 and 25°C.

### Milk fat separation and processing

The results obtained for the raw milk used for milk fat extraction, using the standard methods for dairy products, can be observed in Table 1. As expected, the lyophilized fat layer, in theory corresponding to the total dry extract of the milk's fat layer, is composed mainly of lipids (>80%) but also contains small amounts of protein and lactose. This combination of different substances enables the production of unique nano-delivery systems with tremendous potential for oral administration of active molecules.

**Table 1 - Physicochemical characterization of milk fat layer, regarding total dry extract, total fat, total protein, total ash and lactose. The dry milk fat layer values were calculated using the total dry extract results of the milk fat layer. Results shown as mean ± standard deviation, n = 3. This data was obtained for milk collected from dairy cows.**

| | Total dry extract (%m/m) | Total fat (%m/m) | Total protein (%m/m) | Total ash (%m/m) | Lactose (%m/m) |
|---|---|---|---|---|---|
| Milk fat layer | 37 ± 1 | 31 ± 2 | 2.4 ± 0.1 | 0.5 ± 0.1 | 3.6 ± 0.7 |
| Dry milk fat layer | | 83 ± 4 | 6.4 ± 0.2 | 1.4 ± 0.1 | 9.6 ± 1.8 |

The almost 10% of lactose (dry matter basis) could raise some biocompatibility issues, particular with lactose intolerance consumers. However, this potential limitation can be mitigated by obtaining the fat layer from lactosefree milk instead of raw milk (i.e., non-lactose free).

### Milk fat globule-based nanocarriers characterization

The developed nanocarriers were primarily characterized using DLS. The results obtained for the comparison of nanocarriers produced by the ultrasonication (MiGloN_S) and HPH (MiGloN_H) methods are shown in Figure 1.

As shown in Figure 1, the produced nanocarriers possess two distinct populations: a smaller and more abundant population, with approximately 150 nm and accounting for 80% of all the carriers in the formulation; and a larger population of approximately 550 nm that accounts for only 20% of carriers. These two populations can be observed regardless of the production method used, ultrasonication or HPH, and also present similar dimensions within each population. The latter indicates that regardless of the used production method, the nanocarrier properties are maintained and highlight the easy scalability of production. Both production methods yielded formulations with polydispersity indexes of 0.27, indicating that despite having two distinct populations each of them can be considered reasonably monodisperse. Furthermore, the dimensions of both populations are compatible with the known intestinal uptake mechanisms for lipid nanoparticles [2, 3], which points out their potential to address absorption and bioavailability issues. The developed nanocarriers also present a high negative surface charge (-27 mV) indicating that they will be less likely to aggregate during storage, improving the over-time stability of the dispersion.

The nanocarriers produced using caprine milk fat layer via the sonication-based method presented different sizes than those using bovine milk. In fact, caprine milk-based nanocarriers only presented one population of around 120 nm in diameter indicating that a difference in lipid composition can considerably affect nanocarrier dimensions. Different milk sources did not however affect the zeta potential value, perhaps indicating that the outer layers of the nanocarriers are less influenced by animal source. This study was performed to evaluate if the milk fat globule-based nanocarriers (MiGloN) production could be scaled. To do so MiGloN produced on a lab-scale via the sonication-based method were compared with nanocarriers obtained using a high-pressure homogenization-based method, which was selected due to it widespread use in the food industry. The results reported enable the conclusion that the production of MiGloN is robust regardless of the method used. For this reason, all of the following results are regarding MiGloN produced via the standard sonication-based method using bovine milk unless expressly stated otherwise.

### Lyophilization

The nanocarriers were subjected to lyophilization to determine if they could be converted and manipulated in a dry solid state without compromising their properties. The lyophilization was carried with and without the addition of a cryoprotector (AEROSIL ^{®}) and the resuspension process was attempted with and without ultrasound assistance to assess the optimal procedure (Figure 2).

It can be observed that the lyophilization process does not appear to alter the nanocarriers' properties, as after resuspension they are similar to the original nanocarriers (Figure 2). Furthermore, the results show that the addition of AEROSIL or the ultrasound-assisted resuspension produces similar effects to the formulations without either of these steps. The latter indicates that, if lyophilization is required or beneficial, the process can be performed without any additional concern, either in the preparation or resuspension steps, which can significantly reduce the overall cost and duration of the freeze-drying process.

### Shelf-life stability

The MiGloN formulations were stored at RT and periodically characterized to evaluate their shelf-life stability and determine for how long they remained viable under the storage conditions. The characterization was performed with DLS regarding the hydrodynamic diameter, polydispersity index and zeta potential over 300 days, results shown in Figure 3.

The results indicate that even after 300 days of storage at RT the nanocarriers appear unaltered when considering their size and size distribution profile. Some slight variations appear to occur in the larger and less abundant population (Figure 3, black squares) but this should not compromise the efficacy of the formulation. The zeta potential values appeared to decrease, in modulus, after 20 days of storage, probably due to a conformational shift in the nanocarrier matrix, and afterwards seemed to stabilize. Regardless, across all tested timepoints, all values are sufficiently high, in modulus, as not to influence the formulations' stability. A similar study was conducted for nanocarriers obtained with the sonication method and using caprine milk fat layer. These results showed that the caprine-based nanocarriers could resist storage for up to 210 days, at room temperature, indicating that the MiGloN stability does not appear to be altered when milk from a different animal is used.

### Gastric resistance

The MiGloN formulations were incubated with gastric mimetic medium to evaluate if they could resist digestion in this organ of the digestive tract. To properly assess the effects of this medium three timepoints were compared: ti - nanocarriers after production; t0 - nanocarriers with contact with gastric mimetic medium; and tf - nanocarriers incubated for 2 h in gastric mimetic medium (Figure 4).

The results show that in contact with gastric mimetic medium there is a tendency to a small increase in the diameter of the larger population, most likely due to the adsorption of medium constituents, such as pepsin, to the surface of the nanocarriers. It can also be seen that no alterations in size occur in either of the populations after the 2 h of incubation, indicating that the nanocarriers appear to resist digestion in gastric conditions.

### Intestinal resistance

The MiGloN formulations were incubated with intestinal mimetic medium to evaluate if they could resist digestion in this organ of the digestive tract. To properly assess the effects of this medium three timepoints were compared: ti - nanocarriers after production; t0 - nanocarriers with contact with intestinal mimetic medium; and tf - nanocarriers incubated for 4 h in intestinal mimetic medium (Figure 5).

It can be observed that there is an increase in the diameter of the larger population of nanocarriers, in accordance with the tendency observed in the gastric resistance assay and, again, most likely due to the adsorption of medium components. Once again, it can also be seen that no alterations in size occur in either of the populations after the 4 h of incubation, indicating that the nanocarriers appear to resist digestion in intestinal conditions. To confirm these results and ensuring that the lipases did not destroy only some of the nanocarriers, they were also analyzed using NTA (Table 2), as it is also a quantitative measurement, unlike DLS.

**Table 2 - Nanoparticle tracking analysis results for milk fat globule-based nanocarriers (MiGloN) after production (tᵢ), after contact with intestinal medium (t₀) and after 4 h incubation in intestinal medium (t_{f}). n = 3. This data was obtained for the nanocarriers obtained with the sonication method and using bovine milk fat layer.**

| | Mean (nm) | Mode (nm) | SD (nm) | Concentration (particle/mL) |
|---|---|---|---|---|
| tᵢ MiGloN | 224.0 | 136.4 | 97.7 | 4.17 e+8 ± 1.1 e+7 |
| t₀ MiGloN | 190.7 | 123.1 | 85.1 | 1.56 e+8 ± 1.1 e+7 |
| t_{f} MiGloN | 192.4 | 155.9 | 86.8 | 1.84 e+8 ± 1.1 e+7 |

These results confirm that intestinal conditions do not alter the nanocarriers properties, as can be seen when comparing the mean and mode diameters across all timepoints. Furthermore, the nanocarrier concentration (Table 2) also does not change after the incubation period supporting the idea that they can resist digestion in intestinal conditions.

### Ruminal resistance

The MiGloN formulations produced with a sonication-based method and using caprine fat layer were submitted to a ruminal resistance assay to evaluate if they could resist digestion in this organ of the bovine digestive tract. To properly assess the effects of this medium two timepoints were compared: ti - nanocarriers with contact with ruminal fluid; and tf - nanocarriers incubated for 24 h in ruminal fluid (Figure 10).

The ruminal medium possesses a high number of microorganisms, some of which with dimensions similar to the nanocarriers. The results, however, show that there is a clear difference in dimensions when the nanocarriers are added or not, indicating that the protocol used can effectively separate the nanocarriers from the microbiota. Moreover, the results clear indicate that even after 24 h of incubation in ruminal conditions the nanocarriers remained unaltered and should therefore be resistant to digestion in this organ. This shows a tremendous potential MiGloN produced with caprine milk fat for rumen-bypass delivery systems.

### Encapsulation potential

To evaluate the potential of the MiGloN to deliver active agents, vitamin D, vitamin E and DHA were selected for encapsulation, due to their lipophilic nature and their importance in nutrition. The %EE and %LC of vitamin D and DHA obtained using the developed nanocarriers are found in Table 3, at this time a quantification of vitamin E was not possible and, therefore, these results are not shown.

**Table 3 - Encapsulation efficiency (%EE) and loading capacity (%LC) of vitamin D, vitamin E and DHA in the milk fat globule-based nanocarriers. Values shown as mean ± standard deviation, n = 3. This data was obtained for the nanocarriers obtained with the sonication method and using bovine milk fat layer.**

| | %EE (%) | %LC (%) |
|---|---|---|
| Vitamin D | 100 ± 1 | 10.20 ± 0.02 |
| Vitamin E | - | - |
| DHA | 64 ± 23 | 6.4 ± 2.3 |

The nanocarriers presented a very high encapsulation potential both for vitamin D (100% of added amount) and DHA rendering an also high %LC values, with the encapsulated agent accounting for more than 6% of the overall nanocarrier mass (Table 3). These high values can be explained by the highly lipophilic nature of these molecules. As shown previously, the milk fat layer used to produce the nanocarriers possessed more than 80% fat, which facilitates the incorporation of lipophilic molecules. Furthermore, when considering vitamin D the amount of active agent encapsulated is a thousand fold higher than the recommended daily supplement dose [32]. This enables the nanocarriers to be used as vitamin D nanodelivery vehicles in either the food and feed industries or biomedical applications. However, the quantification of DHA and vitamin E was complicated, and these methods should be revised to ensure a correct and reliable assessment of their encapsulation potential within the MiGloN. It should also be noted, that as comparison between the nanocarriers produced using bovine or caprine milk fat layer, vitamin D was also encapsulated into the latter and similar results were obtained. This confirms that nanocarriers have great potential for the encapsulation of lipophilic molecules regardless of animal selected as the milk's source.

### Cytotoxicity assay

The MTT assay was used to determine the potential cytotoxicity of the developed MiGloN (Figure 6), according to the existing regulations [33]. The results indicated that the nanocarriers, with or without vitamin D encapsulation, did not present any cytotoxicity in any of the tested concentrations (at least up to 2.5 mg/mL of milk fat).

The results obtained not only confirmed the safety of the developed nanocarrier, but also proved that the combination and structure of the selected components in nanocarrier form also did not present any toxicity. The results indicated a considerably low toxicity in fibroblasts, at least up to 2.5 mg/mL, making them safe for use as biomaterials in foods, feeds or biomedical devices according to the existing regulations, which state that an effect or condition is only considered toxic when it reduces viability by 30% or more [33]. These results indicate that the proposed nanocarriers are not inherently toxic and could potentially be used in humans or monogastric animals, however in vivo studies should still be performed to ensure their safety in actual use.

### Hemolysis assay

The hemolytic potential of the MiGloN was also evaluated using fresh human red blood cells (Figure 7), to determine if, once in the bloodstream, they could be harmful to its cells.

It can be seen that, for all of the concentrations tested, the MiGloN never differ from the negative control indicating that they do not possess any hemolytic activity. These results were expected, considering the biocompatibility results previously obtained but this assay enables the conclusion that once in the bloodstream the MiGloN would not be harmful or any way impair the crucial role that blood, in particular red blood cells, play in living organisms.

### Antioxidant capacity assays

The antioxidant capacity was evaluated using the ABTS radical scavenging assay, which enables a good comparison of antioxidants of different capacities, and the ORAC assay, which is a highly biorelevant assay due to the nature of the radicals used. The results obtained for both these assays can be observed in Figures 8 and 9, and Table 4.

**Table 4 - Antioxidant assay results, expressed as µmol Trolox equivalents (TxEq) per mg of nanocarriers, for both the ABTS radical scavenging and the ORAC assay performed with vitamin E loaded (MiGloN_E) and unloaded milk fat globule-based nanocarriers (MiGloN). Values shown as mean ± standard deviation, n = 3. This data was obtained for the nanocarriers obtained with the sonication method and using bovine milk fat layer.**

| | ABTS assay TxEq (µmol/µg) | ORAC assay TxEq (µmol/µg) |
|---|---|---|
| MiGloN | 0.114 | 0.165 |
| MiGloN_E | 0.197 | 0.499 |

The results show that vitamin E has a dose dependent antioxidant capacity and both assays indicated that the encapsulation of vitamin E within the MiGloN increased their activity. This is expected, since due to the chemical structure of vitamin E it is likely that it would be located in the outer layers of the nanocarriers, thus remaining available to exert its antioxidant effects. However, and more interestingly it can be observed that MiGloN themselves present some antioxidant activity both for ABTS and the oxygen species radicals indicating that even without the encapsulation of antioxidant agents they could exert antioxidant effects. This hypothesis is supported by the fact the MiGloN with encapsulated vitamin E presents superior activity to that of either of the components by themselves and that they most work in synergy to achieve their antioxidant capacity. Furthermore, by observing the Trolox equivalence (Table 4) it can be seen that the MiGloN seem to be more effective at scavenging radical oxygen species than the ABTS radical, most likely due to the superior size of the latter that may not interact so easily with nanocarriers. However, since oxygen radicals are more biologically relevant this may not impact their potential applications.

In this work milk-fat derived nanocarriers - MiGloN - were successfully produced using simple and easily scalable methods that rely on equipment that is already used in the food industry. These nanocarriers showed excellent physical properties for oral applications and were found to be highly stable, both in the shelf during storage and in the gastrointestinal tract after consumption. Furthermore, they demonstrated great in vitro biocompatibility and no hemolytic activity whatsoever. They were also able to encapsulate several active molecules, namely vitamin D, vitamin E and DHA, highlighting their potential as nano delivery systems. In fact, it was found that the MiGloN themselves possessed antioxidant activity, which further supports their tremendous potential for applications in both health and nutrition. Lastly but not less important, these extraordinary nanocarriers are composed of milk fat which brings an added value to dairy products and advances towards a circular economy approach for the development of nutraceuticals and therapies.

### Experimental data

### 1. Materials

Vitamin D3 (cholecalciferol), vitamin E (a-tocopherol), Docosahexaenoic acid (DHA), Tween^{®}60, ethylenediaminetetraacetic (EDTA) acid disodium salt dihydrate, dimethyl sulfoxide (DMSO), Trolox, 2,20-azinobis-(3-ethylbenzothiazoline-6-sulphonate) (ABTS), 2,2'-Azobis(2-amidinopropane) dihydrochloride (AAPH), fluorescein sodium salt, 3-(4,5-dimethylthiazol-2-yl)-2,5-diphenyltetrazolium bromide (MTT), Tritontm X-100, pepsin and pancreatin (from porcine pancreas) were purchased from Sigma-Aldrich (St. Luis, MO, USA). Dulbecco's Modified Eagle's Medium (DMEM), penicillin-streptomycin (10000 U/mL) mix, fetal bovine serum (FBS) and amphotericin B (Fungizone) were purchased from Gibco^{®} (Invitrogen Corporation, UK). Aqueous solutions were prepared with double-deionized water (Arium Pro, Sartorius AG, Göttingen, Germany). Raw milk was obtained from three multiparous adult Holstein cows, not pregnant, that were fitted with a ruminal cannula (10 cm diameter; Bar Diamond Inc., Parma, ID, previously approved and licensed by the Portuguese Directorate-General of Food and Veterinary Medicine of the Ministry for Agriculture and Sea, permit 0421/000/000/2015). The cows were housed at the Vairão Agricultural Campus of School of Medicine and Biomedical Sciences, University of Porto (Vila do Conde, Portugal). Cows were handled in strict accordance with good animal practice as defined by the national authority and European Union Directive 2010/63/EU. Experimental animal procedures were approved by the Local Animal Ethics Committee of School of Medicine and Biomedical Sciences, University of Porto, licensed by the Portuguese Directorate-General of Food and Veterinary Medicine of the Ministry for Agriculture and Sea, and conducted by trained scientists (FELASA category C). This study was also performed in accordance with ARRIVE guidelines. Each cow was fed a total mixed ration, based on corn silage or haylage, with fresh drinking water always available. Goat milk was kindly provided by a local goat farmer.

### 2. Methods

### 2.1 Milk fat globule-based nanocarriers production

### 2.1.1 Milk fat separation and processing

Raw milk was centrifuged at 14000 xg for 30 min at 4 °C, to separate the milk cream from the remaining milk matrix. The milk cream was then frozen overnight at -80 °C (Deep Freezer GFL^{®}, Burgwedel, Germany) and afterwards lyophilized using a laboratory freeze drier (LyoQuest -85 plus v.407, Telstar ^{®} Life Science Solutions, Terrassa, Spain) for 72 h at -80 °C under a pressure of 40 Pa. The resulting milk fat layer was then sealed with parafilm and stored at -20 °C until further use.

The separated milk fat, prior to freeze-drying, was also characterized using standard methods for dairy products at "Associação Interprofessional do Leite e Laticínios" (accredited by the Portuguese Accreditation Institute, IPAC, in accordance with NP EN ISO/IEC 17025:2018) to determine total fat, total protein, total ash, lactose and total dry extract.

### 2.1.2 Sonication-based method

An organic-solvent free method based on ultrasounds, using the previously processed milk fat layer and Tween60 as surfactant, was used to produce nanocarriers [34]. In short, 150 mg of milk fat and 50 mg surfactant were placed at 55 °C in a water bath for 10 min. Afterwards, 4.5 mL of double deionized water (ddH₂O), heated at the same temperature, were added to the previous mixture. The resulting emulsion was sonicated using a probe-type sonicator for 5 min at 80% amplitude (model VCX-130 with a VC 18 probe, Sonic & Materials Inc., Newtown, CT, USA) to form a nanoemulsion and afterwards allowed to cool down at RT between 20 and 25°C, rendering an aqueous suspension of nanocarriers, which was stored at RT, between 20 and 25°C, until further use.

To evaluate the potential of the nanocarriers for nanodelivery applications, as a proof-of-concept, 20 mg of selected active agents were added to the fat and surfactant mixture. The agent can be added either prior to or after the heating step, depending on the agent's properties and stability. The active agents considered were: folic acid, vitamin D, vitamin E and DHA (a long chain polyunsaturated fatty acid - LC-PUFA). The remaining procedure was followed as described above.

### 2.1.3 High-pressure homogenization production method

An organic-solvent free method based on high-pressure homogenization (HPH) [11], using the previously processed milk fat layer and Tween60 as surfactant, was used to produce nanocarriers. In short, 1500 mg of milk fat and 500 mg surfactant were placed at 55 °C in a water bath for 10 min. Afterwards, 45 mL of ddH₂O, heated at the same temperature, were added to the previous mixture. The resulting emulsion was pre-homogenized, for 5 min at 12000 rpm (T25 digital, ULTRA TURRAX^{®} - IKA^{®}-Werke GmbH & Co. KG, Staufen, Germany), and then kept at 55 °C in a water bath until further processing. Using a 10 mL syringe, 9 mL of the previous emulsion were placed in the HPH chamber (SPCH-10-EP Ultra High-Pressure Homogenizer, Stansted Fluid Power Products Ltd, Stansted, UK) and homogenized at 350 MPa and 55 °C. The resulting nanoemulsions were collected and allowed to cool at RT to enable nanocarrier formation. The nanocarrier dispersions were placed in glass vials and stored at RT, between 20 and 25°C, until further usage.

### 2.2 Characterization of the Milk fat globule-based nanocarriers

### 2.2.1 Diameter and Zeta potential

The nanocarrier formulations were characterized in terms of mean diameter and size distribution profile (polydispersity index - PdI) by dynamic light scattering (DLS) using a 90Plus Particle Size Analyzer (Brookhaven Instruments Corporation, Holtsville, NY, USA) and in terms of zeta potential by electrophoretic light scattering using a ZetaPALS Zeta Potential Analyzer (Brookhaven Instruments Corporation, Holtsville, NY, USA). A wavelength of 660 nm, a temperature of 20 °C, and a detection angle of 90° were used in the measurements with a refractive index 1.33 [34]. All samples were diluted using ddH₂O.

Detailed nanoparticle size distribution was also determined using nanoparticle tracking analysis (NTA) equipped with a 488 nm laser and a high sensitivity scientific CMOS camera (NanoSight NS300, Malvern Instruments, Worcestershire, UK). Samples were diluted at a ratio of 1:100,000, injected using a 1 mL sterile syringe in the viewing chamber and measured under constant flow (infusion rate of 50) at 24 °C. Size measurements were obtained based on a 20 s video captured with a camera level of 10 and a detection threshold of 5. For each sample, 5 size measurements were conducted. Data were analysed using the NTA 3.4. software [35].

### 2.2.2 Lyophilization

Firstly, 2 mL of nanocarrier dispersion, supplemented with 1% AEROSIL^{®} and without any cryoprotector addition, were frozen overnight at -80 °C (Deep Freezer GFL^{®}, Burgwedel, Germany) and were afterwards lyophilized using a laboratory freeze drier (LyoQuest -85 plus v.407, Telstar ^{®} Life Science Solutions, Terrassa, Spain) for 72 h at -80 °C under a pressure of 40 Pa. The lyophilized nanocarriers were resuspended, with and without ultrasound assistance, in the same volume of ddH₂O (2 mL) and characterized in terms of size, PdI and zeta potential as previously described.

### 2.2.3 Shelf-life stability

To evaluate the shelf-life of the developed nanocarrier formulations, they were stored at RT and assessed for their size, size distribution profile and zeta potential after 1, 7, 14, 21, 28, 44, 60, 90, 120, 150, 180, 240 and 300 days, using the previously described methods.

### 2.2.4 Stability in gastric conditions

The gastric medium was mimicked using simulated gastric fluid prepared according to INFOGEST guidelines [36], supplemented with pepsin at 1 mg/mL. Nanocarriers were incubated, in triplicate, in the previously mentioned medium, at 37 °C, under light stirring. The same medium without nanocarriers was also incubated and used as control, also in triplicate. Aliquots were collected from each replica at 0 h (*t0*) and at 2 h (*tf*) of incubation for further analysis. Both samples and controls were assessed in terms of size using DLS and compared with the values obtained for nanocarriers without any contact with the gastric mimicking medium (*ti*).

### 2.2.5 Stability in intestinal conditions

The intestinal medium was mimicked using simulated intestinal fluid prepared according to INFOGEST guidelines [36], supplemented with pancreatin at 3 mg/mL. Nanocarriers were incubated, in triplicate, in the previously mentioned medium, at 37 °C, under light stirring. The same medium without nanocarriers was also incubated and used as control, also in triplicate. Aliquots were collected from each replica at 0 h (*t0*) and at 4 h (*tf*) of incubation for further analysis. Both samples and controls were assessed in terms of size using DLS and compared with the values obtained for nanocarriers without any contact with the intestinal mimicking medium (*ti*).

### 2.2.6 Stability in ruminal conditions

Ruminal contents were obtained from three multiparous (number of parity = 2) adult Holstein cows, dry and not pregnant that were fitted with a ruminal cannula (10 cm diameter; Bar Diamond Inc., Parma, ID, previously approved and licensed by the Portuguese Directorate-General of Food and Veterinary Medicine of the Ministry for Agriculture and Sea, permit 0421/000/000/2015). The cows were housed at the Vairão Agricultural Campus of School of Medicine and Biomedical Sciences, University of Porto (Vila do Conde, Portugal). Cows were handled in strict accordance with good animal practice as defined by the national authority and European Union Directive 2010/63/EU. Experimental animal procedures were approved by the Local Animal Ethics Committee of School of Medicine and Biomedical Sciences, University of Porto, licensed by the Portuguese Directorate-General of Food and Veterinary Medicine of the Ministry for Agriculture and Sea, and conducted by trained scientists (FELASA category C). This study was also performed in accordance with ARRIVE guidelines. Each cow was fed a total mixed ration, based on corn silage or haylage, and always had fresh drinking water available. Ruminal contents were collected before the morning meal from the 4 quadrants of the rumen and placed in a 4 L pre-warmed (39 °C) thermal jug. In the laboratory, the ruminal digesta of each cow was homogenized and strained through 4 layers of linen cloth at 39 °C in O₂-free CO₂ atmosphere. The interval between the collection of the ruminal contents and incubation never exceeded 60 min. One part of the strained ruminal fluid, pH 6.3, was diluted anaerobically into 4 parts of Kansas State Buffer 48 and mixed at 39 °C in an O₂-free CO₂ atmosphere. Thirty milliliters (25 mL of buffered ruminal fluid and 5 mL of each SLN formulation) were dispensed anaerobically into 125 mL serum bottles (Sigma-Aldrich Inc., St. Louis, MO, USA) containing 250 mg (dry matter) of wheat straw, sealed with butyl rubber stoppers and aluminum crimp caps (Sigma-Aldrich Inc., St. Louis, MO, USA), and incubated in a water bath at 39 °C. Each MiGloN formulation, as well as blanks containing no MiGloN, were incubated in quadruplicate. Two of these replicates of each sample and blanks were placed in an ice bath immediately after the addition of the MiGloN formulation to serve as negative controls (t0 samples, where t represents the time and 0 indicates that no incubation occurred). Fermentations were stopped after 24 h by cooling the bottles in an ice-slurry bath (tf samples, where t represents the time and f indicates that the incubation occurred until the end of the designated time period). The samples were subsequently compared with MiGloN that did not contact the rumen inoculum (ti samples, where t represents time and i represents the MiGloN characterized after production).

The contents of the flasks were transferred to Falcon tubes and centrifuged at 500 xg for 5 min to separate any remaining feed and the heaviest microorganisms, such as protozoa, from the MiGloN. The supernatant was collected and centrifuged at 30000 xg for 20 min to separate the MiGloN from the bacteria and other microorganisms that did not deposit in the first centrifugation. Both supernatants and deposits were collected for characterization in terms of size, PdI and zeta potential. This procedure was also performed on rumen inoculum and buffer mixture without the addition of any SLN (blanks), and these results were used as controls to verify the separation of the MiGloN and rumen inoculum.

### 2.2.7 Biocompatibility

The L929 mouse fibroblastic cell line was selected for the in vitro biosafety characterization of the nanocarriers, as they are recommended for biomaterial safety evaluation for biomedical devices and food applications [33].

Cells were cultivated in DMEM, supplemented with 10% (v/v) FBS, 1% (v/v) penicillin/streptomycin mixture and 1% (v/v) amphotericin B, at 37 °C in 5% CO₂ atmosphere. The medium was replaced every three days and when cells were confluent, they were detached from the culture flasks with a scrapper (NuncTM Cell Scrappers, Thermofisher Scientific; Waltham, MA USA). After this physical detachment, cells were centrifuged at 300 xg for 5 min in a HeraeusTM MultifugeTM X1R centrifuge (Thermo Fisher Scientific; Waltham, USA) and then resuspended in fresh DMEM. Counting of viable cells was performed using a Neubauer chamber (Improved Neubauer Bright-Line, Boeco; Germany) in a Motic^{®} AE2000 Binocular Inverted Microscope (Motic Electric Group Co., Ltd; Xiamen, Fujian, China) [11].

The effect of the nanocarriers on cell viability was evaluated using the resazurin assay [35]. In short, cells were seeded at a density of 1 x10⁴ cells per well in 96-well plates and cultured in DMEM-F12 medium supplemented with 10% (v/v) FBS and 1% (v/v) streptomycin/gentamycin in a humidified incubator (37°C, 5% CO₂) for 24 h. Culture medium was then removed and replaced with different concentrations of each nano formulation, rendering final concentrations of 0.5, 0.75, 1, 1.25, 1.5, 2, 2.25 and 2.5 mg of milk fat per mL of medium. After the incubation period, the medium was removed and replaced by fresh culture medium containing 10% (v/v) of resazurin. After incubation for 4 h in the dark (37 °C, 5% CO₂), the fluorescence of resorufin (λex = 560 nm; λem = 590 nm) was measured (Spectramax Gemini EM spectrofluorometer). Cells incubated with only culture medium were used as positive control. The results were normalized and compared with the positive controls (in theory, 100% of viability).

### 2.2.8 Hemolytic assay

To evaluate the hemolytic potential of the nanoformulations and their suitability for an intravenous administration, a hemolysis assay was performed as described by Granja et al. [37]. In short, human blood from 3 healthy donors was collected in EDTA-coated tubes, centrifuged at 955 xg for 5min at 4°C (Allegra X-15R Centrifuge, Beckman Coulter, Fullerton, California) to separate the red blood cells (RBCs) from the remaining blood components and washed three times with physiological saline solution (0.85%). Nanocarriers was then diluted in saline solution and incubated with the RBCs (4% v/v in saline solution) in a 96-well plate at 37 °C for 1h. Saline solution and Triton X-100 (1%) were used as negative and positive control, respectively. After the incubation period, the supernatant was collected and the amount of released hemoglobin was quantified by UV-vis spectrophotometry at 540nm in a microplate reader (Biotek Instruments, Winooski, VT, USA). The percentage of hemolysis was calculated as follows (equation 1): $Hemolysis \left(%\right) = \frac{Abs \left(sample\right) - Abs \left(negative control\right)}{Abs \left(positive control\right) - Abs \left(negative control\right)} \times 100$ (1)

All the procedures were carried out according to the principles of the declaration of Helsinki. Blood samples were kindly donated by Serviço de Hematologia from Centro Hospitalar Universitário do Porto - Hospital de Santo António. (Porto, Portugal).

### 2.2.9 Encapsulation potential

The encapsulation efficiency of vitamin D (%EE_VD) and DHA (%EE_DHA) was determined as the difference between the total amount of active agent added in the preparation of nanocarriers and the amount of agent outside the nanocarrier (equation 2). The percentage of agent in the nanocarrier composition, loading capacity (%LC), was also calculated (equation 3). $% EE \left(%\right) = \frac{Total amount of agent - amount of agent in the supernatant}{Total amount of agent} \times 100$ (2) $% LC \left(%\right) = \frac{Amount of encapsulated agent}{Amount of fat layer + Amount of surfactant} \times 100$ (3)

Firstly, the nanocarriers suspensions were diluted 10x and filtrated with Amicon^{®} Ultra Centrifugal Filters Ultracell-50 kDa (EMD Millipore, Darmstadt, Germany) at 4000 xg for 20 min using an Allegra^{®} X-15R centrifuge (Beckman Coulter; Pasadena, CA, USA). The supernatant was then collected and quantified using UV/vis-photospectrometry, for vitamin D the absorbance was measured at 270 nm and the solvent used as 10% ethanol in ddH₂O and for DHA the absorbance was measured at 237 nm and solvent was absolute ethanol. A quantification of vitamin E was not performed at this time due to separation issues.

### 2.2.10 Antioxidant assays

The antioxidant capacity of the formulations was assessed using the ABTS radical scavenging and the oxygen radical absorbance capacity (ORAC) assays, which were selected for their selectivity and biological relevance. A calibration curve was developed with Trolox standards, and the results are also expressed as µmol Trolox equivalents per mg of nanocarriers.

### 2.2.11 ABTS radical scavenging assay

The ABTS solution was prepared mixing equal volumes of 7 mM ABTS and 2.45 mM potassium persulfate, separately prepared in water, and left incubating overnight at room temperature protected from light. For sample analysis, the ABTS solution was diluted with the double deionized water to an absorbance of 0.90 ± 0.02 at 734 nm, and 100 µL of nanocarriers suspensions and free vitamin E solutions were added to 100 µL of diluted ABTS solution. After 15 min of incubation protected from light, SynergyTM HT Multimode plate reader (BioTekfi Instruments Inc., Winooski, VT, USA) was used to measure sample absorbance. A sample blank was tested in each assay, all determinations were carried out in triplicate, and the percentage of radical scavenging activity was determined by comparing the absorbance of the samples in relation to the controls.

### 2.2.12 ORAC assay

The reaction was carried out at 40°C in 75 mM phosphate buffer (pH 7.4) and the final assay mixture (200 µL) contained fluorescein (70 nM), AAPH (14 mM), and antioxidant (Trolox or samples). The fluorescence was recorded during 140 min (reading once every min) and the total area under the curve was afterwards calculated. A SynergyTM HT Multimode plate reader (BioTekfi Instruments Inc., Winooski, VT, USA) was used to measure fluorescence, with 485 nm excitation and 520 nm emission filters. Black polystyrene 96-well microplates were used. AAPH and Trolox solutions were prepared daily, and fluorescein was diluted from a stock solution (1.17 mM) in 75 mM phosphate buffer (pH 7.4). All reaction mixtures were prepared in triplicate.

### 2.2.13 Statistical analysis

All statistical analyses were performed with IBM SPSS Statistics (SPSS 27.0, Armonk, NY, USA). For the data that followed a normal distribution, parametric tests were performed. Statistical differences between two groups were calculated based on unpaired t-test and for three or more groups were calculated based on one-way ANOVA, followed by Tukey's multiple comparisons test. When the data did not follow a normal distribution, non-parametric test was performed. Statistical differences between two groups were calculated based on unpaired Mann-Whitney test and for three or more groups were calculated based in Kruskall-Wallis analysis. Data are expressed as mean ± standard deviation (SD), and p-values of < 0.05 were considered statistically significant.

### Bibliography

1. Pinheiro, A., et al., Extracellular vesicles: intelligent delivery strategies for therapeutic applications. Journal of Controlled Release, 2018. 289: p. 56-69.
2. Managuli, R.S., et al., Targeting the intestinal lymphatic system: a versatile path for enhanced oral bioavailability of drugs. Expert Opinion on Drug Delivery, 2018. 15(8): p. 787-804.
3. Talegaonkar, S. and A. Bhattacharyya, Potential of Lipid Nanoparticles (SLNs and NLCs) in Enhancing Oral Bioavailability of Drugs with Poor Intestinal Permeability. AAPS PharmSciTech, 2019. 20(3): p. 121.
4. Mehryab, F., et al., Exosomes as a next-generation drug delivery system: An update on drug loading approaches, characterization, and clinical application challenges. Acta Biomaterialia, 2020. 113: p. 42-62.
5. Patil, N.A. and B. Kandasubramanian, Functionalized polylysine biomaterials for advanced medical applications: A review. European Polymer Journal, 2021. 146: p. 110248.
6. Cieslik, M., K. Nazimek, and K. Bryniarski, Extracellular Vesicles-Oral Therapeutics of the Future. International Journal of Molecular Sciences, 2022. 23(14): p. 15.
7. Hsu, C.Y., et al., Use of Lipid Nanocarriers to Improve Oral Delivery of Vitamins. Nutrients, 2019. 11(1): p. 1-30.
8. Dobrucka, R. and M. Ankiel, Possible applications of metal nanoparticles in antimicrobial food packaging. Journal of Food Safety, 2019. 39(2): p. e12617.
9. Resende, D., S.A. Costa Lima, and S. Reis, Nanoencapsulation approaches for oral delivery of vitamin A. Colloids and Surfaces B: Biointerfaces, 2020. 193: p. 111121.
10. Tang, C.H., Assembled milk protein nano-architectures as potential nanovehicles for nutraceuticals. Advances in Colloid and Interface Science, 2021. 292: p. 25.
11. Albuquerque, J., et al., Production of rumen- and gastrointestinal-resistant nanoparticles to deliver lysine to dairy cows. Scientific Reports, 2023. 13(1): p. 16667.
12. Gelaye, Y., Application of nanotechnology in animal nutrition: Bibliographic review. Cogent Food & Agriculture, 2024. 10(1): p. 2290308.
13. Date, A.A., J. Hanes, and L.M. Ensign, Nanoparticles for oral delivery: Design, evaluation and state-of-the-art. Journal of Controlled Release, 2016. 240: p. 504-526.
14. Dumont, C., et al., Lipid-based nanosuspensions for oral delivery of peptides, a critical review. International Journal of Pharmaceutics, 2018. 541(1-2): p. 117-135.
15. Gordillo-Galeano, A. and C.E. Mora-Huertas, Solid lipid nanoparticles and nanostructured lipid carriers: A review emphasizing on particle structure and drug release. European Journal of Pharmaceutics and Biopharmaceutics, 2018. 133: p. 285-308.
16. Roos, C., et al., Mechanistic modelling of intestinal drug absorption - The <i>in vivo</i> effects of nanoparticles, hydrodynamics, and colloidal structures. European Journal of Pharmaceutics and Biopharmaceutics, 2018. 133: p. 70-76.
17. Donahue, N.D., H. Acar, and S. Wilhelm, Concepts of nanoparticle cellular uptake, intracellular trafficking, and kinetics in nanomedicine. Advanced Drug Delivery Reviews, 2019. 143: p. 68-96.
18. Lin, T.T., et al., Bioactives in bovine milk: chemistry, technology, and applications. Nutrition Reviews, 2021. 79(Suppl 2): p. 48-69.
19. Mohan, M.S., et al., Milk fat: opportunities, challenges and innovation. Critical Reviews in Food Science and Nutrition, 2021. 61(14): p. 2411-2443.
20. Singh, K.S., et al., Bio-therapeutics from human milk: prospects and perspectives. J Appl Microbiol, 2021. 131(6): p. 2669-2687.
21. Xie, S., et al., Potential uses of milk proteins as encapsulation walls for bioactive compounds: A review. Journal of Dairy Science, 2022. 105(10): p. 7959-7971.
22. Kimpel, F. and J.J. Schmitt, Review: Milk Proteins as Nanocarrier Systems for Hydrophobic Nutraceuticals. J Food Sci, 2015. 80(11): p. R2361-6.
23. Zempleni, J., et al., Milk-Derived Exosomes and Metabolic Regulation. Annu Rev Anim Biosci, 2019. 7: p. 245-262.
24. Costa, C., et al., Proteins Derived from the Dairy Losses and By-Products as Raw Materials for Non-Food Applications. Foods, 2021. 10(1): p. 11.
25. Queiros, M.D., et al., Milk Fat Modification Strategies for Technological Application on a Macro, Micro and Nanoscale: A Review. Food Reviews International, 2021: p. 19.
26. Queirós, M.d.S., et al., Development of solid lipid nanoparticle and nanostructured lipid carrier with dairy ingredients. International Dairy Journal, 2022. 130: p. 105186.
27. Zarif, B., et al., Potential of milk fat globule membrane's phospholipids and anhydrous milk fat based nanostructured lipid carriers for enhanced bioaccessibility of vitamin D3. International Dairy Journal, 2023. 147: p. 105766.
28. Venkat, M., L.W. Chia, and T.T. Lambers, Milk polar lipids composition and functionality: a systematic review. Crit Rev Food Sci Nutr, 2022: p. 1-45.
29. Dingler, A. and S. Gohla, Production of solid lipid nanoparticles (SLN): scaling up feasibilities. Journal of Microencapsulation, 2002. 19(1): p. 11-16.
30. Guerin, J., et al., Milk fat globule membrane glycoproteins: Valuable ingredients for lactic acid bacteria encapsulation? Critical Reviews in Food Science and Nutrition, 2019. 59(4): p. 639-651.
31. Baer, R.J., et al., Composition and Properties of Milk and Butter from Cows Fed Fish Oil1. Journal of Dairy Science, 2001. 84(2): p. 345-353.
32. Institute of Medicine Committee to Review Dietary Reference Intakes for Vitamin, D. and Calcium, The National Academies Collection: Reports funded by National Institutes of Health, in Dietary Reference Intakes for Calcium and Vitamin D, A.C. Ross, et al., Editors. 2011, National Academies Press (US) Copyright © 2011, National Academy of Sciences.: Washington (DC).
33. International Organization for Standardization, ISO 10993-5:2009 - Biological evaluation of medical devices - Part 5: Tests for in vitro cytotoxicity. 2009: Geneva.
34. Albuquerque, J., et al., Applying nanotechnology to increase the rumen protection of amino acids in dairy cows. Scientific Reports, 2020. 10(1): p. 6830.
35. Granja, A., et al., Mitoxantrone-loaded lipid nanoparticles for breast cancer therapy - Quality-by-design approach and efficacy assessment in 2D and 3D in vitro cancer models. International Journal of Pharmaceutics, 2021. 607: p. 121044.
36. Brodkorb, A., et al., INFOGEST static in vitro simulation of gastrointestinal food digestion. Nature Protocols, 2019. 14(4): p. 991-1014.
37. Granja, A., et al., Folate receptor-mediated delivery of mitoxantrone-loaded solid lipid nanoparticles to breast cancer cells. Biomedicine & Pharmacotherapy, 2022. 154: p. 113525.

## Claims

1. A formulation comprising milk fat globule-based nanocarriers for the delivery of active agents, comprising:
- between 60% to 66% of lipid content on a dry basis m/m;
- between 4% to 5% of protein content on a dry basis m/m;
- between 23% to 27% of a non-ionic surfactant on a dry basis m/m;
and further comprising at least one type of active agent encapsulated in the nanocarriers.

2. The formulation according to the previous claim, wherein the active ingredient, or mixtures thereof, is present between 5% to 20% on a dry basis m/m.

3. The formulation according to any of the previous claims, wherein it further comprises between 6% to 8% of lactose on a dry basis m/m.

4. The formulation according to any of the previous claims, wherein at least one active agent is selected from bioactive compounds, nutraceuticals, therapeutic agents, dietary supplements, or mixtures thereof.

5. The formulation according to any of the previous claims, wherein the lipid content comprises lipids selected from short-chain saturated fatty acids, short-chain unsaturated fatty acids, long-chain saturated fatty acids, long-chain unsaturated fatty acids, triacylglycerols, diacylglycerols, monoacylglycerols, phosphatidylcholine, phosphatidylethanolamine, phosphatidylinositol, phosphatidylserine, glycerophospholipids, phospholipids, sphingomyelin, sphingolipids, cholesterol, or mixtures thereof.

6. The formulation according to any of the previous claims, wherein the protein content comprises proteins selected from mucin, xanthine dehydrogenase/oxidase, butyrophilin, cluster of differentiation CD36, Periodic Acid Schiff 6/7, Periodic Acid Schiff III, adipophilin, fatty-acid binding proteins, immunoglobulins, proteins derived from the cytoplasm of secretory-epithelial cells, skim milk constituents, proteins from milk leucocytes, or mixtures thereof.

7. The formulation according to any of the previous claims, wherein the nanocarriers comprise 75% to 90% nanocarriers of an average particle size between 160 and 220 nm, and 10% to 25% of an average particle size between 500 and 600 nm.

8. The formulation according to any of the claims 1 to 6, wherein the nanocarriers have an average particle size between 80 and 140 nm.

9. The formulation according to any of the previous claims, wherein the nanocarriers have an average -25 mV to -35 mV Zeta potential.

10. The formulation according to any of the previous claims, wherein the milk fat is obtained from milk of a mammal selected from bovine, dairy cow, goat, sheep, donkey, camel, human, or mixtures thereof.

11. The formulation according to any of the previous claims, for oral, topical, transdermal or intravenous administration.

12. The formulation according to any of the previous claims, for use in a food product, a nutritional supplement, an enteral or parenteral assisted feeding, a cosmetic composition, or a pharmaceutical composition.

13. Method to obtain the milk fat globule-based nanocarriers of any of the previous claims,
comprising the following steps:
- Obtaining milk from a mammal source;
- Centrifuging the milk between 13000 and 15000 xg for a time between 27 and 33 min at a temperature between 4 and 20 ⁰C, to separate the milk cream from the remaining milk components;
- Freezing the milk cream overnight between -75 and -85 °C;
- Lyophilizing the milk cream for a time between 10 to 20 h at a temperature between -70 to -85 °C under a pressure between 500 to 20000 Pa, to obtain milk fat;
- Adding the milk fat and a non-ionic surfactant in a ratio between 2.5:1 and 3.5:1 of milk fat:surfactant, in a water bath;
- Adding an active agent, or mixtures thereof, in a proportion between 1:5 and 1:20 of active agent:milk fat+surfactant;
- Heating the mixture at a temperature between 45 and 55 °C for a time between 10 and 15 mins;
- Adding double deionised water heated at the same temperature in a proportion between 18:1 and 20:1 of water:dry components, to produce an emulsion; and:
- Sonicating the emulsion for a time between 4 and 6 min at 75% and 85% amplitude, to form a nanoemulsion;
- Cooling the nanoemulsion to a temperature between 20 and 25°C, to obtain an aqueous suspension comprising the nanocarriers;
or
- Homogenizing the emulsion for a time between 4 and 6 mins at between 11000 and 13000 rpm,
- Keeping the emulsion in a water bath at a temperature between 45 and 55 °C;
- Transferring the emulsion to a syringe and placing it in a high-pressure homogenization chamber and homogenizing at a pressure between 340 and 360 MPa and temperature between 45 and 55 °C;
- Collecting the resulting nanoemulsion and allowing to cool at a temperature between 20 and 25°C, to obtain an aqueous suspension comprising the nanocarriers.

14. Method according to the previous claim, wherein the non-ionic surfactant is selected from Tween60, Tween80, polyvinyl alcohol, Poloxamers, Fatty acid esters of glycerol or sorbitan, lecithin, saponins or Amino acid-based surfactants.

## Patentansprüche

1. Formulierung, die auf Milchfettkügelchen basierende Nanotransporter zur Verabreichung von Wirkstoffen umfasst, bestehend aus:
- einem Lipidgehalt von 60 % bis 66 % auf Trockenbasis (m/m);
- einem Proteingehalt von 4 % bis 5 % auf Trockenbasis (m/m;
- einen Gehalt an nichtionischem Tensid von 23 % bis 27 % auf Trockenbasis (m/m);
und weiterhin mindestens eine Art von Wirkstoff umfassen, der in den Nanoträgern eingekapselt ist.

2. Formulierung nach dem vorstehenden Anspruch, wobei der Wirkstoff oder Mischungen davon in einem Anteil von 5 % bis 20 % auf Trockenbasis (m/m) vorliegt.

3. Formulierung nach einem der vorstehenden Ansprüche, wobei sie ferner zwischen 6% und 8% Laktose, bezogen auf die Trockenmasse (m/m), enthält.

4. Formulierung nach einem der vorstehenden Ansprüche, wobei mindestens ein Wirkstoff aus bioaktiven Verbindungen, Nutraceuticals, therapeutischen Wirkstoffen, Nahrungsergänzungsmitteln oder Mischungen daraus ausgewählt ist.

5. Formulierung nach einem der vorstehenden Ansprüche, wobei der Lipidgehalt Lipide umfasst, die ausgewählt sind aus kurzkettigen gesättigten Fettsäuren, kurzkettigen ungesättigten Fettsäuren, langkettigen gesättigten Fettsäuren, langkettigen ungesättigten Fettsäuren, Triacylglycerolen, Diacylglycerolen, Monoacylglycerolen, Phosphatidylcholin, Phosphatidylethanolamin, Phosphatidylinositol, Phosphatidylserin, Glycerophospholipide, Phospholipide, Sphingomyelin, Sphingolipide, Cholesterin oder Mischungen davon umfasst.

6. Formulierung nach einem der vorstehenden Ansprüche, wobei der Proteingehalt Proteine umfasst, die aus Mucin, Xanthindehydrogenase/Oxidase, Butyrophilin und dem Differenzierungscluster CD36 ausgewählt sind, Periodsäure-Schiff-6/7, Periodsäure-Schiff-III, Audiophilen, Fettsäure-bindende Proteine, Immunglobuline, aus dem Zytoplasma von sekretorischen Epithelzellen stammende Proteine, Bestandteile von Magermilch, Proteine aus Milchleukozyten oder Mischungen davon umfasst.

7. Formulierung nach einem der vorstehenden Ansprüche, wobei die Nanoträger 75% bis 90% Nanoträger mit einer durchschnittlichen Partikelgröße zwischen 160 und 220 nm sowie 10% bis 25% mit einer durchschnittlichen Partikelgröße zwischen 500 und 600 nm umfassen.

8. Formulierung nach einem der Ansprüche 1 bis 6, wobei die Nanoträger eine durchschnittliche Partikelgröße zwischen 80 und 140 nm aufweisen.

9. Formulierung gemäß einem der vorstehenden Ansprüche, wobei die Nanoträger ein durchschnittliches Zeta-Potential von -25 mV bis -35 mV aufweisen.

10. Formulierung nach einem der vorstehenden Ansprüche, wobei das Milchfett aus Milch eines Säugetiers gewonnen wird, das aus Rindern, Milchkühen, Ziegen, Schafen, Eseln, Kamelen, Menschen oder Mischungen davon ausgewählt ist.

11. Formulierung nach einem der vorstehenden Ansprüche zur oralen, topischen, transdermalen oder intravenösen Verabreichung.

12. Formulierung nach irgendeinem der vorhergehenden Ansprüche zur Verwendung in einem Lebensmittelprodukt, einem Nahrungsergänzungsmittel, einer enteralen oder parenteral unterstützten Ernährung, einer kosmetischen Zusammensetzung oder einer pharmazeutischen Zusammensetzung.

13. Verfahren zur Herstellung der auf Milchfettkügelchen basierenden Nanoträger nach einem der vorhergehenden Ansprüche, dass die folgenden Schritte umfasst:
- Gewinnung von Milch aus einer Säugetierquelle;
- Zentrifugieren der Milch bei 13.000 bis 15.000 xg über einen Zeitraum von 27 bis 33 Minuten bei einer Temperatur zwischen 4 und 20 °C, um den Milchrahm von den übrigen Komponenten der Milch zu trennen;
- Einfrieren des Milchrahm über Nacht bei einer Temperatur zwischen -75 und - 85 °C;
- Gefriertrocknen des Milchfetts über einen Zeitraum von 10 bis 20 Stunden bei einer Temperatur zwischen -70 und -85 °C unter einem Druck zwischen 500 und 20.000 Pa, um Milchfett zu gewinnen;
- Zugabe des Milchfetts und eines nichtionischen Tensids im Verhältnis von 2,5:1 bis 3,5:1 von Milchfett : Tensid in einem Wasserbad;
- Zugabe eines Wirkstoffs oder von Mischungen daraus in einem Verhältnis von 1:5 bis 1:20 von Wirkstoff : Milchfett + Tensid;
- Erhitzen der Mischung auf eine Temperatur zwischen 45 und 55 °C für eine Dauer zwischen 10 und 15 Minuten;
- Zugabe von doppelt deionisiertem Wasser, das auf dieselbe Temperatur erhitzt wurde, in einem Verhältnis zwischen 18:1 und 20:1 von Wasser:Trockenkomponenten
, um eine Emulsion herzustellen; und:
- Beschallung der Emulsion über einen Zeitraum von 4 bis 6 Minuten bei einer Amplitude von 75% bis 85%, um eine Nanoemulsion zu bilden;
- Abkühlen der Nanoemulsion auf eine Temperatur zwischen 20 und 25 °C, um eine wässrige Suspension zu erhalten, welche die Nanoträger enthält;
oder
- Homogenisieren der Emulsion für eine Dauer zwischen 4 und 6 Minuten bei einer Drehzahl zwischen 11.000 und 13.000 U/min,
- Halten der Emulsion in einem Wasserbad bei einer Temperatur zwischen 45 und 55 °C;
- Überführen der Emulsion in eine Spritze und Einbringen in eine Hochdruckhomogenisierungskammer sowie Homogenisieren bei einem Druck zwischen 340 und 360 MPa und einer Temperatur zwischen 45 und 55 °C;
- Sammeln der resultierenden Nanoemulsion und Abkühlen auf einer Temperatur zwischen 20 und 25°C, um eine wässrige Suspension zu erhalten, welche die Nanoträger enthält.

14. Verfahren nach dem vorhergehenden Anspruch, wobei das nichtionische Tensid ausgewählt ist aus Tween60, Tween80, Polyvinylalkohol, Poloxameren, Fettsäureestern von Glycerin oder Sorbitan, Lecithin, Saponinen oder Tensiden auf Aminosäurebasis.

## Revendications

1. Formulation comprenant des nanovecteurs à base de globules de matière grasse du lait pour l'administration d'agents actifs, comprenant :
- entre 60 % et 66 % de teneur en lipides sur base sèche m/m ;
- entre 4 % et 5 % de teneur en protéines sur base sèche m/m ;
- entre 23 % et 27 % d'un tensioactif non ionique sur base sèche m/m ;
et comprenant en outre au moins un type d'agent actif encapsulé dans les nanovecteurs.

2. Formulation selon la revendication précédente, dans laquelle l'ingrédient actif, ou des mélanges de celui-ci, est présent entre 5 % et 20 % sur base sèche m/m.

3. Formulation selon l'une quelconque des revendications précédentes, dans laquelle comprend en outre entre 6 % et 8 % de lactose sur base sèche m/m.

4. Formulation selon l'une quelconque des revendications précédentes, dans laquelle au moins un agent actif est choisi parmi des composés bioactifs, des nutraceutiques, des agents thérapeutiques, des compléments alimentaires, ou des mélanges de ceux-ci.

5. Formulation selon l'une quelconque des revendications précédentes, dans laquelle la teneur en lipides comprend des lipides choisis parmi des acides gras saturés à chaîne courte, des acides gras insaturés à chaîne courte, des acides gras saturés à chaîne longue, des acides gras insaturés à chaîne longue, des triacylglycérols, des diacylglycérols, des monoacylglycérols, la phosphatidylcholine, la phosphatidyléthanolamine, le phosphatidylinositol, la phosphatidylsérine, des glycérophospholipides, des phospholipides, la sphingomyéline, des sphingolipides, le cholestérol, ou des mélanges de ceux-ci.

6. Formulation selon l'une quelconque des revendications précédentes, dans laquelle la teneur en protéines comprend des protéines choisies parmi la mucine, la xanthine déshydrogénase/oxydase, la butyrophiline, le cluster de différenciation CD36, l'acide périodique de Schiff 6/7, l'acide périodique de Schiff III, l'adipophiline, des protéines de liaison aux acides gras, des immunoglobulines, des protéines dérivées du cytoplasme des cellules épithéliales sécrétoires, des constituants du lait écrémé, des protéines provenant des leucocytes du lait, ou des mélanges de celles-ci.

7. Formulation selon l'une quelconque des revendications précédentes, dans laquelle les nanovecteurs comprennent 75% à 90% de nanovecteurs d'une taille moyenne de particule comprise entre 160 et 220 nm, et 10% à 25% d'une taille moyenne de particule comprise entre 500 et 600 nm.

8. Formulation selon l'une quelconque des revendications 1 à 6, dans laquelle les nanovecteurs ont une taille moyenne de particule comprise entre 80 et 140 nm.

9. Formulation selon l'une quelconque des revendications précédentes, dans laquelle les nanovecteurs ont un potentiel zêta moyen de -25 mV à -35 mV.

10. Formulation selon l'une quelconque des revendications précédentes, dans laquelle la matière grasse du lait est obtenue à partir de lait d'un mammifère choisi parmi un bovin, une vache laitière, une chèvre, un mouton, un âne, un chameau, un humain, ou des mélanges de ceux-ci.

11. Formulation selon l'une quelconque des revendications précédentes, pour une administration orale, topique, transdermique ou intraveineuse.

12. Formulation selon l'une quelconque des revendications précédentes, pour une utilisation dans un produit alimentaire, un supplément nutritionnel, une alimentation assistée entérale ou parentérale, une composition cosmétique, ou une composition pharmaceutique.

13. Procédé d'obtention des nanovecteurs à base de globules de matière grasse du lait selon l'une quelconque des revendications précédentes, dans lequel il comprend les étapes suivantes :
- Obtenir du lait à partir d'une source mammifère ;
- Centrifuger le lait entre 13000 et 15000 xg pendant une durée comprise entre 27 et 33 min à une température comprise entre 4 et 20 °C, pour séparer la crème de lait des autres composants du lait restants ;
- Congeler la crème de lait pendant la nuit entre -75 et -85 °C ;
- Lyophiliser la crème de lait pendant une durée comprise entre 10 et 20 h à une température comprise entre -70 et -85 °C sous une pression comprise entre 500 et 20000 Pa, pour obtenir de la matière grasse du lait ;
- Ajouter la matière grasse du lait et un tensioactif non ionique selon un rapport compris entre 2,5:1 et 3,5:1 de matière grasse du lait:tensioactif, dans un bain-marie ;
- Ajouter un agent actif, ou des mélanges de celui-ci, selon une proportion comprise entre 1:5 et 1:20 d'agent actif:matière grasse du lait+tensioactif ;
- Chauffer le mélange à une température comprise entre 45 et 55 °C pendant une durée comprise entre 10 et 15 min ;
- Ajouter de l'eau doublement désionisée chauffée à la même température selon une proportion comprise entre 18:1 et 20:1 d'eau:composants secs, pour produire une émulsion ; et :
- Soniquer l'émulsion pendant une durée comprise entre 4 et 6 min à 75% et 85% d'amplitude, pour former une nanoémulsion ;
- Refroidir la nanoémulsion à une température comprise entre 20 et 25 °C, pour obtenir une suspension aqueuse comprenant les nanovecteurs ;
ou
- Homogénéiser l'émulsion pendant une durée comprise entre 4 et 6 min à entre 11000 et 13000 tr/min,
- Maintenir l'émulsion dans un bain-marie à une température comprise entre 45 et 55 °C ;
- Transférer l'émulsion dans une seringue et la placer dans une chambre d'homogénéisation à haute pression et homogénéiser à une pression comprise entre 340 et 360 MPa et à une température comprise entre 45 et 55 °C ;
- Recueillir la nanoémulsion obtenue et la laisser refroidir à une température comprise entre 20 et 25 °C, pour obtenir une suspension aqueuse comprenant les nanovecteurs.

14. Procédé selon la revendication précédente, dans lequel le tensioactif non ionique est choisi parmi le Tween60, le Tween80, l'alcool polyvinylique, les poloxamères, les esters d'acides gras du glycérol ou du sorbitane, la lécithine, les saponines ou les tensioactifs à base d'acides aminés.
